## Europäisches Patentamt

## European Patent Office

## Office européen des brevets

(19)

(11) Veröffentlichungsnummer: **0 133 488**
**B1**

(12) **EUROPÄISCHE PATENTSCHRIFT**

(45) Veröffentlichungstag der Patentschrift:
**11.03.87**

(51) Int. Cl.⁴: **A 61 B 6/03**

(21) Anmeldenummer: **84108358.7**

(22) Anmeldetag: **16.07.84**

(54) **Computertomograph.**

(30) Priorität: **29.07.83 DE 3327707**

(43) Veröffentlichungstag der Anmeldung:
**27.02.85 Patentblatt 85/9**

(45) Bekanntmachung des Hinweises auf die Patenterteilung:
**11.03.87 Patentblatt 87/11**

(84) Benannte Vertragsstaaten:
**DE FR**

(56) Entgegenhaltungen:
**DE - A - 3 017 494**
**FR - A - 2 385 374**
**FR - A - 2 387 634**
**FR - A - 2 391 697**
**FR - A - 2 433 333**
**GB - A - 2 004 436**

(73) Patentinhaber: **Siemens Aktiengesellschaft Berlin und München, Wittelsbacherplatz 2, D-8000 München 2 (DE)**

(72) Erfinder: **Rauch, Werner, Saarlouiser Strasse 45, D-8500 Nürnberg (DE)**
Erfinder: **Schmitt, Günter, Forststrasse 16, D-8520 Erlangen (DE)**
Erfinder: **Tschunt, Edgar, Im Winkel 9, D-8521 Rathsberg (DE)**

## Beschreibung

Die Erfindung betrifft einen Computertomographen mit einer Messeinheit aus einer Röntgenstrahlenquelle und einem Strahlenempfänger, bei der die Röntgenstrahlenquelle ein fächerförmiges Röntgenstrahlenbündel aussendet, das auf dem aus einer Reihe von Einzeldetektoren bestehenden, ringförmigen, das Messfeld umschliessenden Strahlenempfänger auftrifft, von denen jeder ein der empfangenen Strahlenintensität entsprechendes elektrisches Signal bildet, bei dem Mittel zur Drehung des Röntgenstrahlenbündels um das Aufnahmeobjekt zur Durchstrahlung einer in der Fächerebene liegenden Schicht des Aufnahmeobjektes unter verschiedenen Richtungen vorgesehen sind, bei dem eine Messwertverarbeitungseinheit vorhanden ist, der die Ausgangssignale der Einzeldetektoren, die bei den verschiedenen Durchstrahlungsrichtungen erzielt werden, zugeführt werden und die daraus die Schwächungswerte vorbestimmter Punkte in der durchstrahlten Ebene des Aufnahmeobjektes bestimmt, und bei dem eine Bildwiedergabevorrichtung zur bildlichen Wiedergabe der berechneten Schwächungswerte vorhanden ist, wobei jeder Einzeldetektor derart verstellbar gelagert ist, dass er aus einer Lage ausserhalb des Röntgenstrahlenbündels in eine Lage gebracht werden kann, in der er vom Röntgenstrahlenbündel getroffen wird.

Bei einem bekannten Computertomographen dieser Art (vgl. FR-A-2 385 374) ist der Strahlenempfänger kardanisch gelagert und der derjenige Teil, der zur Erfassung der aus dem Aufnahmeobjekt austretenden Röntgenstrahlung erforderlich ist, wird jeweils in das Röntgenstrahlenbündel mit Hilfe von Führungsmitteln eingeschwenkt. Dadurch ergibt sich eine Taumelbewegung des Strahlenempfängers und die Einzeldetektoren werden jeweils nicht senkrecht von Röntgenstrahlung getroffen. Ferner sind die bewegten Massen relativ gross, was insbesondere dann störend ist, wenn kurze Abtastzeiten angestrebt werden, für die eine schnelle Taumelbewegung erforderlich ist.

Bei einem weiteren bekannten Computertomographen der eingangs genannten Art (vgl. FR-A-2 391 697) ist jeder Einzeldetektor verstellbar gelagert, so dass er wahlweise in das Röntgenstrahlenbündel hineinbewegt oder aus diesem herausbewegt werden kann. Dadurch ist zwar eine Taumelbewegung des gesamten Strahlenempfängers vermieden; der konstruktive Aufwand für die Verstellung der Einzeldetektoren ist jedoch sehr gross.

Der Erfindung liegt die Aufgabe zugrunde, einen Computertomographen der eingangs genannten Art so auszubilden, dass bei gegenüber dem Stand der Technik verringertem konstruktivem Aufwand unter Vermeidung einer Taumelbewegung des Stahlenempfängers derjenige Teil des Strahlenempfängers, der in Strahlenrichtung gesehen vor dem Aufnahmeobjekt liegt, in einfacher Weise aus der Röntgenstrahlung entfernt wird.

Diese Aufgabe ist erfindungsgemäss dadurch gelöst, dass die Einzeldetektoren in Detektorgruppen mit gleicher Anzahl von Einzeldetektoren zusammengefasst und dass die Detektorgruppen unabhängig voneinander verstellbar sind. Bei dem erfindungsgemässen Computertomographen werden jeweils diejenigen Detektorgruppen, die in Strahlenrichtung gesehen vor dem Aufnahmeobjekt liegen, so verstellt, dass sie die Röntgenstrahlung nicht stören. Die gruppenweise Verstellung erfordert dabei einen relativ geringen konstruktiven Aufwand.

Eine besonders zweckmässige Lösung zur Verstellung der Detektorgruppen besteht darin, dass eine synchron mit der Drehung des Röntgenstrahlenbündels drehbare Nockenscheibe vorhanden ist, an der Hebel anliegen, die auf die Detektorgruppen einwirken. Diese Hebel können unter Federwirkung stehen, so dass sie mit einem Ende jeweils an die Nockenscheibe gepresst und entsprechend der Kurve auf der Nockenscheibe bewegt werden. Die Steuerung der Detektorgruppen kann kraftschlüssig (Feder) oder formschlüssig (Kulisse oder Doppelkurve) erfolgen.

Die Erfindung ist nachfolgend anhand eines in der Zeichnung dargestellten Ausführungsbeispiels näher erläutert. Es zeigen:

Fig. 1 die für die Erfindung wesentlichen Teile eines Computertomographen nach der Erfindung, und

Fig. 2 eine Einzelheit des Computertomographen gemäss Figur 1 in Seitenansicht.

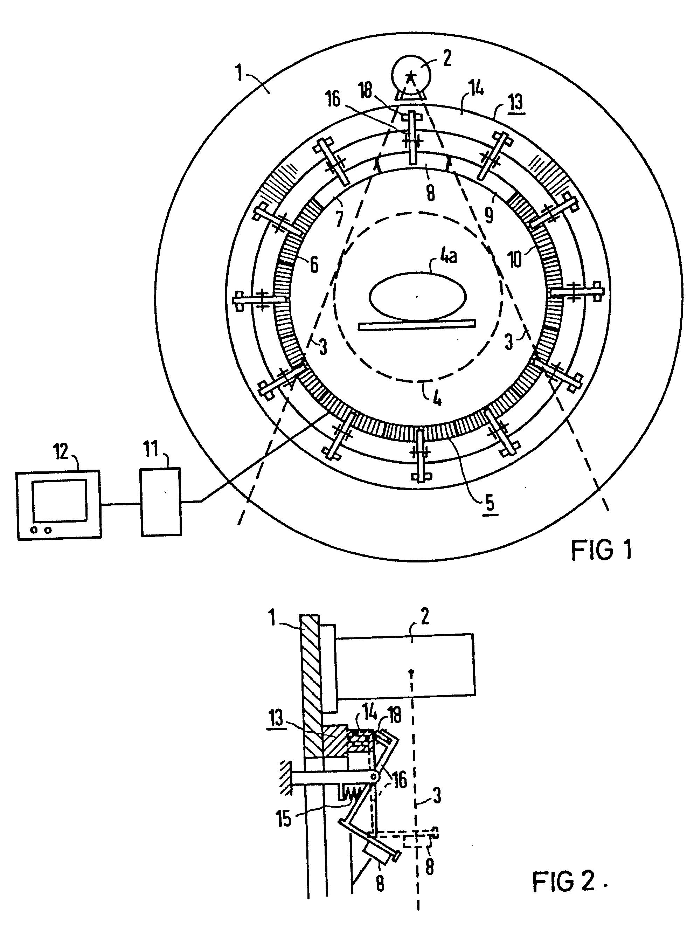

Die Figur 1 zeigt einen Drehring 1, auf dem eine Röntgenstrahlenquelle 2 ortsfest angebracht ist. Die Röntgenstrahlenquelle 2 sendet ein fächerförmiges Röntgenstrahlenbündel 3 aus, dessen Randstrahlen ein kreisförmiges Messfeld 4 bei der Drehung des Drehringes 1 tangieren. Die Messeinheit des dargestellten Computertomographen enthält ausser der Röntgenstrahlenquelle 2 einen Strahlenempfänger 5, der aus einer Reihe von Einzeldetektoren besteht, die das Messfeld ringförmig umschliessen. Die Einzeldetektoren des Strahlenempfängers 5 sind in Detektorgruppen 6, 7, 8, 9, 10 usw. mit gleicher Anzahl von Einzeldetektoren zusammengefasst. Die Detektorgruppen 6 bis 10 sind einzeln unabhängig voneinander senkrecht zur Fächerebene des Röntgenstrahlenbündels 3 in der nachfolgend noch näher beschriebenen Weise verstellbar, so dass jeweils diejenigen Detektorgruppen, die in Strahlenrichtung gesehen vor dem im Messfeld liegenden Aufnahmeobjekt 4a liegen, aus dem Röntgenstrahlenbündel 3 heraus verstellt werden können. Dies sind in der gezeichneten Stellung der Röntgenstrahlenquelle 2 die Detektorgruppen 7, 8, 9. Das Röntgenstrahlenbündel 3 kann demgemäss ungehindert das Aufnahmeobjekt 4a durchdringen und trifft nach seinem Austritt aus dem Aufnahmeobjekt 4a auf den im Röntgenstrahlenbündel liegenden Einzeldetektoren auf. Diese Einzeldetektoren liefern bei den verschiedenen Durchstrahlungsrichtungen, die durch Drehung der Röntgenstrahlenquelle 2 auf dem Drehring 1 erzielt werden, der jeweils empfangenen Strahlenintensität entsprechende elektrische Signale, die einer Messwertverarbeitungseinheit 11 zugeführt werden. Die Messwertverarbeitungseinheit 11 berechnet aus diesen Signalen die Schwächungswerte vorbestimmter Punkte in der durchstrahlten Ebene des Aufnahmeobjektes im Messfeld 4, die auf einem Sichtgerät 12 in Form eines Bildes dieser Ebene wiedergegeben werden.

Zur Verstellung der Detektorgruppen 6 bis 10 usw.

ist auf dem Drehring 1 eine Nockenscheibe 13 vorgesehen, die einen erhabenen Teil 14 aufweist. Der Teil 14 ist von einer stoss- und ruckfreien Kurve für sinusförmigen Bewegungsablauf der gesteuerten Detektorgruppen gebildet. Gegen die Nockenscheibe 13 werden mit Hilfe von Druckfedern, von denen in der Figur 2 eine sichtbar und mit 15 bezeichnet ist, Hebel mit ihren einen Enden gedrückt, die an ihren anderen Enden jeweils eine Detektorgruppe 6 bis 10 usw. tragen. In der Figur 2 sind die Detektorgruppe 8 und der Hebel 16 für diese Detektorgruppe 8 dargestellt. Zur Erfassung der Röntgenstrahlung nehmen die Detektorgruppen 6 bis 10 die in der Figur 2 für die Detektorgruppe 8 gestrichelt gezeichnete Stellung ein. Soll eine der Detektorgruppen, bei dem Beispiel die Detektorgruppen 7 bis 9, von denen in der Figur 2 nur die Detektorgruppe 8 sichtbar ist, aus dem Röntgenstrahlenbündel 3 herausgeschwenkt werden, so erfolgt dies über das Auflaufen einer Rolle 18 am Ende des jeweiligen Hebels 16 auf dem Teil 14 der Kurvenscheibe 13.

Die Detektorgruppen 6 bis 10 usw. und die zugehörigen Hebel nehmen demgemäss in diesem Fall dann die in der Figur 2 für die Komponenten 8, 16 gestrichelt gezeichnete Lage ein.

Das Ausführungsbeispiel zeigt, dass durch geringen konstruktiven Aufwand ein sicheres und schnelles Herausschwenken derjenigen Einzeldetektoren aus dem Röntgenstrahlenbündel 3 erfolgt, die den freien Zugang des Röntgenstrahlenbündels 3 zum Messfeld und damit zum Aufnahmeobjekt behindern würden. Die Erfindung bezieht sich demgemäss auf einen Computermonographen mit Detektorring und ausserhalb dieses Detektorringes um diesen gedrehter Röntgenstrahlenquelle.

## Patentansprüche

1. Computertomograph mit einer Messeinheit (2, 5) aus einer Röntgenstrahlenquelle (2) und einem Strahlenempfänger (5), bei der die Röntgenstrahlenquelle (2) ein fächerförmiges Röntgenstrahlenbündel (3) aussendet, das auf dem aus einer Reihe von Einzeldetektoren bestehenden, ringförmigen, das Messfeld (4) umschliessenden Strahlenempfänger (5) auftrifft, von denen jeder ein der empfangenen Strahlenintensität entsprechendes elektrisches Signal bildet, bei dem Mittel (1) zu Drehung des Röntgenstrahlenbündels (3) um das Aufnahmeobjekt zur Durchstrahlung einer in der Fächerebene liegenden Schicht des Aufnahmeobjektes (4a) unter verschiedenen Richtungen vorgesehen sind, bei dem eine Messwertverarbeitungseinheit (11) vorhanden ist, der die Ausgangssignale der Einzeldetektoren, die bei den verschiedenen Durchstrahlungsrichtungen erzielt werden, zugeführt werden und die daraus die Schwächungswerte vorbestimmter Punkte in der durchstrahlten Ebene des Aufnahmeobjektes bestimmt und bei dem eine Bildwiedergabevorrichtung (12) zur bildlichen Wiedergabe der berechneten Schwächungswerte vorhanden ist, wobei jeder Einzeldetektor derart verstellbar gelagert ist, dass er aus einer Lage ausserhalb des Röntgenstrahlenbündels (3) in eine Lage gebracht werden kann, in der er von dem Röntgenstrahlenbündel (3) getroffen wird, dadurch gekennzeichnet, dass die Einzeldetektoren in Detektorgruppen (6 bis 10 usw.) mit gleicher Anzahl von Einzeldetektoren zusammengefasst sind und dass die Detektorgruppen (6 bis 10 usw.) unabhängig voneinander verstellbar sind, so dass jeweils diejenigen Detektorgruppen (6 bis 10 usw.), die in Strahlenrichtung gesehen vor dem Aufnahmeobjekt (4a) liegen, das Röntgenstrahlenbündel (3) nicht stören.

2. Computertomograph nach Anspruch 1, dadurch gekennzeichnet, dass zur Verstellung der Detektorgruppen (6 bis 10 usw.) eine synchron mit der Drehung des Röntgenstrahlenbündels (3) drehbare Nockenscheibe (13) vorhanden ist, an der Hebel (16) anliegen, die die Detektorgruppen (6 bis 10 usw.) steuern.

## Claims

1. A computerised tomograph comprising a measuring unit (2, 5) consisting of an X-ray source (2) and a radiation receiver (5), wherein the X-ray source (2) transmits a fanshaped X-ray beam (3) which is incident upon the annular radiation receiver (5) which surrounds the measuring field (4) and consists of a row of individual detectors, each of which forms an electric signal which corresponds to the received radiation intensity, means (1) for turning the X-ray beam (3) about the object to be photographed, arranged to irradiate a layer arranged in the fan plane of the object to be photographed (4a) from different directions, a measured value processing unit (11) to which are supplied the output signals obtained in the different directions of irradiation by the individual detectors and which determines therefrom the attenuation value of predetermined points in the irradiated plane of the object to be photographed, and wherein an image reproduction device (12) is arranged for the image reproduction of the calculated attenuation values, where each individual detector is positioned so as to be adjustable such that it can be brought from a position outside the X-ray beam (3) into a position in which the X-ray bundle (3) is incident upon it, characterised in that the individual detectors are combined in detector groups (6 to 10 etc.) having the same number of individual detectors and that the detector groups (6 to 10 etc.) are adjustable independently of one another, so that the detector groups (6 to 10 etc.) which are arranged preceding the object to be photographed (4a) viewed in the direction of radiation, do not disturb the X-ray beam (3).

2. A computerised tomograph as claimed in Claim 1, characterised in that a cam disc (13) is arranged to adjust the detector groups (6 to 10 etc.), which is simultaneously rotatable with the rotation of the X-ray beam (3) and against which rest levers (16) which control the detector groups (6 to 10 etc.).

## Revendications

1. Tomodensitomètre comportant une unité de mesure (2, 5) constituée par une source (2) de rayons X et par un récepteur de rayonnement (5), et dans laquelle la source (2) de rayons X émet un faisceau (3) de rayons X en forme d'éventail, qui tombe sur le récepteur annulaire de rayonnement (5), qui entoure le champ de mesure (4) et est constitué par une série de détecteurs individuels dont chacun forme un signal électrique correspondant à la densité reçue de rayonnement, et dans lequel il est prévu des moyens (1) servant à entraîner en rotation le faisceau (3) de rayons X autour de l'objet de prise de vues pour réaliser l'irradiation d'une couche, située dans le plan de l'éventail du faisceau, de l'objet de prise de vues (4a) suivant des directions différentes, et dans lequel il est prévu une unité (11) de traitement des valeurs de mesure, à laquelle les signaux de sortie des différents détecteurs, qui sont obtenus pour les différentes directions d'irradiation, sont envoyés, et qui détermine à partir de là, les valeurs d'affaiblissement de points prédéterminés situés dans le plan irradié de l'objet de prise de vues, et dans lequel il est prévu un dispositif (12) de reproduction d'images servant à reproduire sous forme imagée les valeurs d'affaiblissement calculées, et dans lequel chaque détecteur individuel est monté de façon à être réglable pour pouvoir être amené depuis une position située à l'extérieur du faisceau (3) de rayon X dans une position dans laquelle ce détecteur est atteint par le faisceau (3) de rayons X, caractérisé par le fait que les détecteurs individuels sont rassemblés par groupes de détecteurs, (6 à 10, etc.) contenant le même nombre de détecteurs individuels, et que les groupes de détecteurs (6 à 10, etc.) peuvent être réglés indépendamment les uns des autres de telle sorte que les groupes de détecteurs (6 à 10, etc.), qui sont situés en avant de l'objet de prise de vues (4a) suivant la direction du rayonnement, ne perturbent pas le faisceau 3) de rayons X.

2. Tomodensitomètre suivant la revendication 1, caractérisé par le fait que pour le réglage des groupes de détecteurs (6 à 10, etc.), il est prévu un disque à came (13) qui peut être entraîné en rotation en synchronisme avec la rotation du faisceau (3) de rayons X et sur lequel prennent appui des leviers (16) qui commandent les groupes de détecteurs (6 à 10, etc.).

FIG 1

FIG 2 .